# FASCICULE DE BREVET EUROPEEN

(11) **EP 1 028 620 B1**
(45) Date de publication et mention de la délivrance du brevet: **23.07.2003**
(21) Numéro de dépôt: 98954535.5
(22) Date de dépôt: 06.11.1998
(51) Int. Cl.: A01H 4/00

(54) **PROCEDE DE RAJEUNISSEMENT DE GYMNOSPERMES PAR EMBRYOGENESE SOMATIQUE**
VERFAHREN ZUR VERJÜNGUNG VON GYMNOSPERM MITTELS SOMATISCHER EMBRYOGENESE
METHOD FOR REJUVENATING GYMNOSPERMS BY SOMATIC EMBRYOGENESIS

(30) Priorité: 10.11.1997 FR 9714105
(43) Date de publication de la demande: 23.08.2000
(73) Titulaire: Afocel, 75008 Paris (FR)
(72) Inventeur: PAQUES, Marc, F-77920 Samois sur Seine (FR); BERCETCHE, Joelle, F-91570 Bièvres (FR)
(74) Mandataire: Grosset-Fournier, Chantal Catherine
(86) Numéro de dépôt international: FR9802381
(87) Numéro de publication internationale: WO99023874

(56) Documents cités:
- WO-A-91/05854
- GB-A- 2 195 656
- US-A- 4 353 184
- US-A- 4 550 528
- US-A- 5 501 972
- DATABASE CABA AN - 8234259 Forest Science (1981) Vol 27 No 2 "Tissue Culture Plantlets produced from Pinus monticola Embryonic materials" Matt,R.L. et al. XP002076590
- DATABASE CABA AN-92113070 HortScience (1990) Vol 25. No 1 "In vitro multiple bud formation by 20-year old western larch buds and stems" Chesick, E. E. et al. XP002076591

## Description

L'invention a pour objet un procédé de rajeunissement de gymnospermes par embryogenèse somatique.

L'invention a pour objet l'utilisation de bourgeons ou d'explants de bourgeons de gymnospermes pour l'initiation de tissus embryogènes à partir de cals embryogènes ou non, et la production de clones de gymnospermes.

L'invention a également pour objet la régénération de gymnospermes, notamment de conifères jeunes, matures ou âgés.

Le brevet US n° 5.501.972 décrit un procédé de production de tissus embryogènes d'épicéa comprenant la culture d'aiguilles ou d'explants provenant d'épicéa, d'environ 26 ans ou moins, pour initier des cals non embryogènes et la culture des cals non embryogènes en présence de cellules nourricières d'épicéa afin de produire des tissus embryogènes, dans lequel les cals non embryogènes et les cellules nourricières sont placés à proximité de chaque côté d'une membrane de filtre.

Ce brevet prétend également divulguer la régénération de plantules d'épicéa. Cependant, il ne donne aucune preuve que des plantules d'épicéa ont été régénérées à partir des tissus embryogènes produits.

A ce jour, il n'existe pas de procédé permettant de cloner des conifères âgés par embryogenèse somatique.

L'un des aspects de l'invention est de proposer un procédé d'initiation de tissus embryogènes de qualité telle qu'il y a effectivement régénération ultérieure de plantes à partir de tissus embryogènes en question.

L'un des aspects de l'invention est également de proposer un procédé de différenciation de cals non embryogènes en tissus embryogènes.

L'un des autres aspects de l'invention est la mise au point d'un procédé de rajeunissement de gymnospermes, notamment de conifères âgés sélectionnés afin d'être clonés et, l'établissement de cultures industrielles d'arbres de haute valeur ajoutée.

L'invention a pour objet l'utilisation de bourgeons en développement ou d'explants de bourgeons en développement, de gymnospermes, notamment de conifères, suite à une reprise de développement naturelle ou non après un arrêt de croissance naturel ou provoqué, pour l'initiation de tissus embryogènes et de cals non embryogènes.

Par tissu embryogène, on désigne un tissu translucide blanc et mucilagineux qui contient des pro-embryons à un stade précoce de développement attachés à des cellules de suspenseurs ; ces masses de tissu mucilagineux s'appellent aussi ESM (embryonal suspensor masses) (amas de suspenseurs et d'embryons).

Par cal non embryogène, on désigne une masse de cellules résultant de la croissance non organisée de cellules végétales généralement produites à partir d'explants en culture.

Grâce à l'invention, il est donc possible d'établir des cultures industrielles d'arbres de haute valeur ajoutée : arbres sélectionnés pour la vigueur, la branchaison, la qualité du bois, la tolérance à des pathogènes et à des conditions climatiques particulières etc...

Les embryons somatiques obtenus par le procédé d'embryogenèse somatique sont considérés comme juvéniles. Etre capable de régénérer des embryons à partir d'arbres âgés signifie donc le rajeunissement, au moins partiel de ceux-ci, et la possibilité de cloner ces arbres après sélection en retrouvant leur vigueur juvénile.

Par "arrêt de croissance", on désigne l'absence de développement des organes et tissus qui constituent le bourgeon ; cet arrêt de croissance résultant de l'arrêt des divisions mitotiques et de l'allongement cellulaire.

Par "en développement", on désigne des bourgeons ou explants dans lesquels l'activité mitotique et/ou l'élongation cellulaire reprennent.

La limite de l'état de reprise du développement est indiqué par l'aspect macroscopique du bourgeon.

Pour fixer les idées, et à titre d'exemple, dans le cas de l'épicéa, l'état de développement suite à une reprise de développement, ne dépasse pas le débourrement des bourgeons, ceux-ci étant sous forme de pinceau arrondi.

L'activité mitotique des tissus peut reprendre 1 mois avant le débourrement chez l'épicéa (A. Hejnowicz, E. Obarska (1995), Structure and development of vegetative buds, from the lower crown of *Picea abies*, Ann Sci For, 52, 433-447).

Selon un mode de réalisation avantageux, l'invention concerne l'utilisation de bourgeons ou d'explants de bourgeons, lesquels explants ou bourgeons sont prélevés sur la période s'étendant de la reprise du développement des bourgeons jusqu'à l'arrêt de croissance de ceux-ci.

De façon avantageuse, les explants ou bourgeons sont prélevés sur la période s'étendant de la reprise d'activité des bourgeons jusqu'à l'arrêt de croissance, et plus particulièrement du gonflement des bourgeons jusqu'à l'élongation de l'axe caulinaire portant le méristème, laquelle élongation est comprise d'environ 0,01 mm à environ 10 mm, et plus particulièrement d'environ 0,05 mm à environ 10 mm.

L'invention concerne l'utilisation de bourgeons ou d'explants de bourgeons dans laquelle les explants de bourgeons sont des aiguilles ou des écailles en cours d'élongation, prélevées sur l'axe caulinaire en élongation.

Un bourgeon est constitué d'un axe caulinaire court portant un ensemble de petites feuilles insérées très près les unes des autres (l'ensemble est appelé "pousse"), terminé par le méristème qui les a engendrées ; le bourgeon en arrêt de croissance étant le plus souvent protégé par des écailles non vivantes.

Chez l'épicéa, les bourgeons terminaux ou latéraux en formation deviennent écailleux puis rentrent dans une phase de repos. Quelques semaines après la période de repos, les bourgeons reprennent leur activité. Ils gonflent et s'allongent. Les bourgeons sont toujours prélevés lorsque l'accroissement en longueur de ceux-ci, après la période de repos, est inférieur ou égal à 10 mm.

Chez les pins, les bourgeons terminaux des rameaux sont considérés au même stade de développement décrit pour l'épicéa. Les bourgeons fasciculaires "dormants" situés à la base des bouquets d'aiguilles portés par les rameaux sont prélevés après leur reprise d'activité, lorsque l'accroissement en longueur est inférieur ou égal à 5 mm.

L'axe caulinaire en élongation est donc l'axe portant les aiguilles en élongation et à son sommet, le méristème. Au cours de la reprise de développement du bourgeon, cette pousse est le siège d'une activité mitotique et/ou d'un allongement cellulaire intense(s) dont résulte l'éclatement du bourgeon et la perte progressive des écailles.

Les aiguilles en élongation sont les feuilles portées par l'axe caulinaire et caractérisées par une activité mitotique et/ou un allongement cellulaire intense(s).

L'invention concerne l'utilisation de bourgeons ou d'explants de bourgeons dans laquelle les conifères sont des gymnospermes appartenant à l'ordre des coniférales, et plus particulièrement aux familles des Pinaceae, Cupressaceae, Taxaceae, Taxodiaceae et plus particulièrement aux genres : Abies, Pinus, Picea, Tsuga, Pseudotsuga, Thyua, Juniperus, Larix, Sequoia.

L'invention concerne également l'utilisation de bourgeons ou d'explants de bourgeons, dans laquelle les aiguilles sont prélevées en dessous du méristème, et au-dessus des premières aiguilles en arrêt d'élongation, et plus particulièrement dans les 2/3 supérieurs de la pousse en élongation, et plus particulièrement dans le 1/3 supérieur de la pousse en élongation.

Par "premières aiguilles en arrêt d'élongation", on désigne les aiguilles pour lesquelles il n'y a plus d'activité mitotique et/ou d'élongation cellulaire.

Par "2/3 supérieurs de la pousse en élongation", on désigne les 2/3 supérieurs de la portion de l'axe caulinaire comprise entre le méristème terminal et les écailles situées à la base du bourgeon.

Par "1/3 supérieur de la pousse en élongation", on désigne le 1/3 supérieur de la portion de l'axe caulinaire compris entre le méristème terminal et les écailles situées à la base du bourgeon.

L'invention a également pour objet l'utilisation de bourgeons ou d'explants de bourgeons, dans laquelle le prélèvement est effectué sur des bourgeons choisis parmi des bourgeons végétatifs terminaux ou latéraux en arrêt de croissance suite à des conditions notamment climatiques naturelles ou artificielles, puis soumis à des conditions de reprise de développement, naturelles ou artificielles.

Par "bourgeons végétatifs terminaux ou latéraux", on désigne respectivement :
- les bourgeons situés au sommet des rameaux ; ils exercent généralement une dominance apicale sur les bourgeons auxiliaires au développement plus réduit ;
- les bourgeons latéraux (axillaires) se développant sur la portion des rameaux située sous le bourgeon terminal ; le développement de ceux-ci peut être amplifié par ablation du bourgeon terminal engendrant la perte de dominance apicale.

Par "conditions climatiques naturelles ou artificielles" (d'arrêt de croissance), on désigne le froid, la sécheresse ou la photopériode résultant des variations saisonnières ou imposées indépendamment de celles-ci en conditions artificielles.

Par "conditions de reprise de développement naturelles ou artificielles", on désigne le froid, la photopériode, la période chaude résultant des variations saisonnières ou imposées indépendamment de celles-ci en conditions artificielles. Outre les facteurs physiques (froid, sécheresse et photopériode) imposés en conditions de culture contrôlées, on peut citer également les traitements par les cytokinines et les gibbérellines.

L'invention a pour objet l'utilisation de bourgeons ou d'explants de bourgeons, dans laquelle les bourgeons sont prélevés sur de l'épicéa et ont la forme d'ananas ou de pinceau arrondi.

L'invention a également pour objet l'utilisation de bourgeons ou d'explants de bourgeons prélevés sur des gymnospermes juvéniles ou matures et, notamment sur des individus âgés d'environ 51 jours à environ 22 ans ou plus depuis la graine et particulièrement d'environ 67 jours à environ 22 ans ou plus, depuis la graine, et notamment d'environ 2 ans à environ 22 ans ou plus depuis la graine.

Par "juvéniles", on entend des individus ayant une croissance et un développement équivalents à ceux d'un semis encore incompétent pour fleurir.

Par "matures", on désigne des plantes âgées ou non, capables de fleurir dans les conditions naturelles favorables.

L'invention concerne l'utilisation de bourgeons ou d'explants de bourgeons, dans laquelle les bourgeons végétatifs sont prélevés sur les plants âgés d'environ 51 jours à environ 22 ans ou plus depuis la graine, notamment d'environ 67 jours à environ 22 ans ou plus depuis la graine, et notamment d'environ 2 ans à 22 ans ou plus depuis la graine et, plus particulièrement, sur les boutures de ces derniers, et plus avantageusement sur les boutures de ceux-ci obtenues par bouturages réitérés tous les trois ans pendant 22 ans ou plus, à partir d'un semis âgé de 4 ans.

L'invention a pour objet l'utilisation de bourgeons ou d'explants de bourgeons, dans laquelle les rameaux sur lesquels sont prélevés les bourgeons sont traités par le froid et notamment soumis à des températures allant d'environ 15°C à environ 0°C pendant environ 10 à environ 20 jours à la lumière ou à l'obscurité, et plus particulièrement soumis à des températures allant d'environ 10°C à environ 5°C pendant environ 15 à environ 5 jours à l'obscurité.

L'invention concerne également le procédé d'initiation de tissus embryogènes et de cals non embryogènes de gymnospermes, notamment de conifères, par mise en culture de bourgeons ou d'explants de bourgeons, notamment d'aiguilles, définis selon l'invention, sur ou dans un milieu d'initiation contenant des substances de croissance utilisées isolément ou en combinaison, plus particulièrement des auxines, des cytokinines, des gibbérellines et plus avantageusement des auxines et des cytokinines.

Parmi les auxines, sont considérés IAA [acide 1H-indole-3 acétique], IBA [acide 1H-indole-3 butanoique], NAA [acide 1-naphtalène acétique], 2,4-D [acide 2,4-dichlorophénoxy acétique], le Picloram [acide 4-amino-3,5,6-trichloropicolinique] ; parmi les cytokinines, la Kinétine [N-(2-furanylméthyl)-1H-purine-6-amine], BAP [N-(phénylméthyl)-lH-purine-6-amine], et 2iP [2-isopentyladénine].

Le milieu d'initiation contient par exemple :
- BAP dont la concentration est comprise entre environ 0 M à environ 2 M, et plus particulièrement d'environ 40 µM à environ 50 µM et plus avantageusement d'environ 25 µM à environ 40 µM;
- NAA dont la concentration est comprise d'environ 0 M à environ 2 M, et plus particulièrement d'environ 50 µM à environ 100 µM et plus avantageusement d'environ 25 µM à environ 40 µM;
- Picloram dont la concentration est comprise d'environ 0 µM à environ 95 µM, et plus particulièrement d'environ 25 µM à environ 40 µM et plus avantageusement d'environ 10 µM à environ 25 µM.

Comme milieu d'initiation, on a recours à un milieu d'initiation classiquement utilisé. A titre d'exemple, sans que cela soit limitatif, on peut citer le milieu de Gupta K. et Durzan D. (Gupta et Durzan, 1986. Plantlet regeneration *via* somatic embryogenesis from subcultured callus of mature embryos of *Picea abies* (Norway spruce), *In vitro* Cell and Develop. Biology 22:685-688).

L'invention a également pour objet le procédé de préparation de tissus embryogènes à partir de cals non embryogènes obtenus par mise en culture de bourgeons ou d'explants de bourgeons selon l'invention, comprenant les étapes suivantes :
- établissement d'une suspension de tissus embryogènes réactifs provenant avantageusement de tissus embryogènes de gymnospermes, et
- co-culture des tissus non embryogènes avec les tissus embryogènes en suspension ou avantageusement immobilisés sur un support sur lequel les tissus pourront s'immobiliser soit par effet mécanique, soit par effet électrochimique ; les tissus non embryogènes étant séparés des tissus embryogènes par une membrane dont la taille moyenne des pores est comprise d'environ 0,20 µm à environ 5,00 µm, avantageusement d'environ 0,23 µm à environ 0,50 µm.

Dans l'établissement de la susdite suspension de tissus embryogènes réactifs, on utilise soit des gymnospermes appartenant à la même espèce que les susdits cals non embryogènes (isoculture), soit des gymnospermes appartenant à une autre espèce (hétéroculture).

Selon un mode de réalisation avantageux, les gymnospermes appartiennent à une espèce plus réactive et différente de celle des cals non embryogènes.

L'expression "tissus embryogènes réactifs" désigne des tissus embryogènes dont la biomasse double au moins après 21 jours de culture dans/sur le milieu d'initiation mentionné ci-dessus.

Par "immobilisés sur un support", on désigne l'accrochage des tissus soit par effet mécanique, soit par effet électrochimique.

Le support utilisé pour l'immobilisation peut être constitué de matériaux naturels (caoutchouc, argile expansée, fibres de cellulose, agar, carraghénanes...) ou artificiels (fibres de verre, polyéthylène, polypropylène, polycarbonate, polyuréthane...) ; les exemples donnés n'étant en aucun cas limitatifs.

Le support consiste avantageusement en une feuille permettant l'immobilisation des tissus par effet mécanique ou électrochimique.

On utilise avantageusement une feuille de polyuréthane réticulée, à base d'un polyol de polyéther à structure cellulaire ouverte et/ou fermée, mais plus avantageusement une feuille de polyuréthane réticulée à cellules ouvertes caractérisées par un nombre de cellules compris de 10 à 60 par 2,54 cm, et plus particulièrement de 20 à 50 par 2,54 cm, et plus avantageusement de 30 à 45 par 2,54 cm, lesquelles cellules sont limitées par des arêtes n'occupant pas plus de 3% du volume total du support. L'épaisseur du support est avantageusement comprise d'environ 0,5 cm à environ 2 cm.

La surface de contact de la ou des membrane(s) isolant les cals non embryogènes des tissus embryogènes réactifs en suspension ou immobilisés dans ou sur un milieu d'induction est comprise d'environ 0,01 fois à environ 0,8 fois la surface totale du milieu de culture, et plus avantageusement d'environ 0,09 fois à environ 0,2 fois.

La membrane séparant les cals non embryogènes des tissus embryogènes réactifs en suspension ou immobilisés dans ou sur un milieu d'induction sera en ester de cellulose, en Téflon® hydrophile, en polycarbonate, en polyéthylène ou en toute autre matière permettant une filtration au travers de pores dont le diamètre est compris d'environ 0,10 µm à environ 5,0 µm et, avantageusement d'environ 0,23 µm à environ 0,50 µm, et plus particulièrement de 0,10 µm à 0,23 µm.

L'étape de préparation des tissus embryogènes, immobilisés ou non, dure environ 1 à environ 10 jours. Pendant la co-culture, les cals non embryogènes croissent et différencient des tissus embryogènes après d'environ 3 à environ 8 mois depuis le début de la co-culture. La biomasse produite par les tissus embryogènes double environ après 21 jours de co-culture, alors que celle des cals non embryogènes est multipliée par un facteur compris environ entre 5 et 6.

Après 21 jours, la totalité du milieu de co-culture est renouvelée par un milieu d'induction frais et le rapport de masse entre les tissus embryogènes et les cals non embryogènes ou évoluant vers l'état embryogène est maintenu d'environ 1,5 à environ 3,5. Les tissus embryogènes sont remplacés tous les 42 jours et le rapport tissus embryogènes/cals non embryogènes est d'environ 1,5 à environ 3,5.

L'invention a pour objet le procédé dans lequel l'étape d'immobilisation a lieu dans un milieu liquide, notamment un milieu de prolifération classique, et l'étape de co-culture a lieu dans un milieu liquide avantageusement différent du susdit milieu liquide utilisé pour l'immobilisation, et notamment un milieu d'induction classique.

L'invention concerne également les tissus embryogènes tels qu'obtenus selon la mise en oeuvre du procédé de l'invention.

On les appellera ci-après "tissus embryogènes initiés".

A l'issue de l'initiation, de façon avantageuse, les tissus embryogènes initiés à partir du matériel végétal décrit précédemment sont stabilisés et préamplifiés en présence de 8 x 10 mg de tissus embryogènes réactifs répartis autour des tissus embryogènes nouvellement initiés, l'ensemble de ces tissus étant cultivé sur le milieu de prolifération classique de Gupta et Durzan (1986) gélifié ou non par de l'agar, de l'agarose, des carraghénanes, du gelrite® (marque déposée par Monsanto) mais avantageusement par de l'agar et/ou du gelrite® et plus particulièrement par de l'agar.

L'invention a également pour objet le procédé de régénération de plantes à partir de tissus embryogènes définis dans l'invention, ou à partir des tissus embryogènes tels qu'obtenus selon l'invention.

Les tissus embryogènes initiés selon le procédé de l'invention ont des caractéristiques telles qu'ils peuvent être régénérés en plantes. En effet, après la stabilisation et la préamplification des tissus embryogènes initiés selon l'invention, on peut régénérer des plantes selon les procédés classiques.

### Description des figures :

### Figure 1 :

Stades de développement et zones définies pour les bourgeons de d'épicéa :
1) repos,
2) activé,
3) débourrement stade 1,
4) débourrement stade 2, et
5) débourrement stade 3.

### Figure 2 :

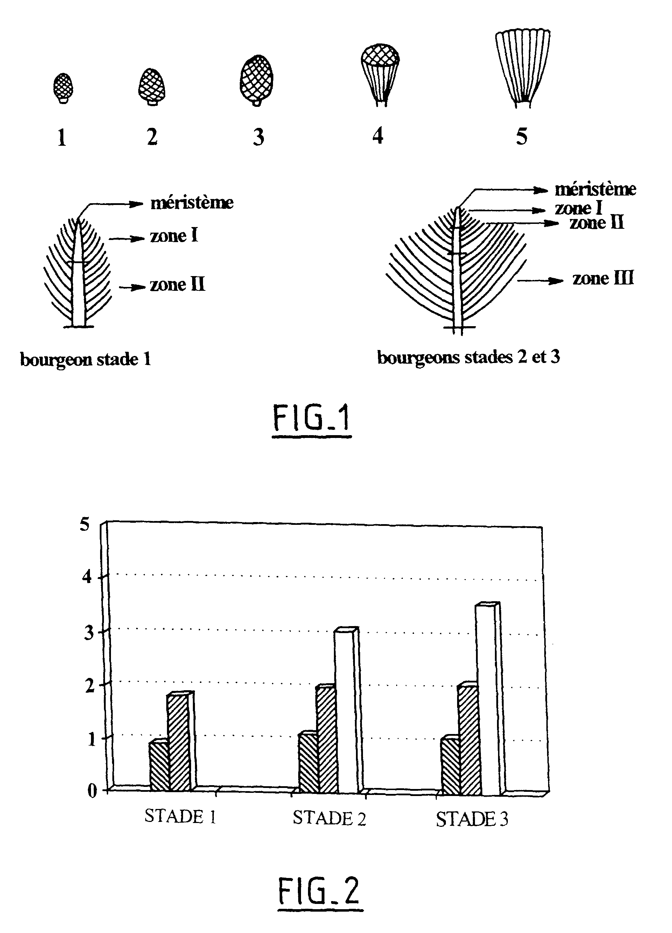

La figure 2 représente la longueur moyenne des aiguilles par zone pour différents stades de développement des bourgeons de *Picea abies*.

En abscisses figurent les stades 1, 2 et 3 de développement de bourgeons.

En ordonnées figure la longueur moyenne des aiguilles en mm.

La colonne hachurée correspond à la zone I. La colonne comportant des traits diagonaux correspond à la zone II et la colonne blanche correspond à la zone III.

### Figure 3 :

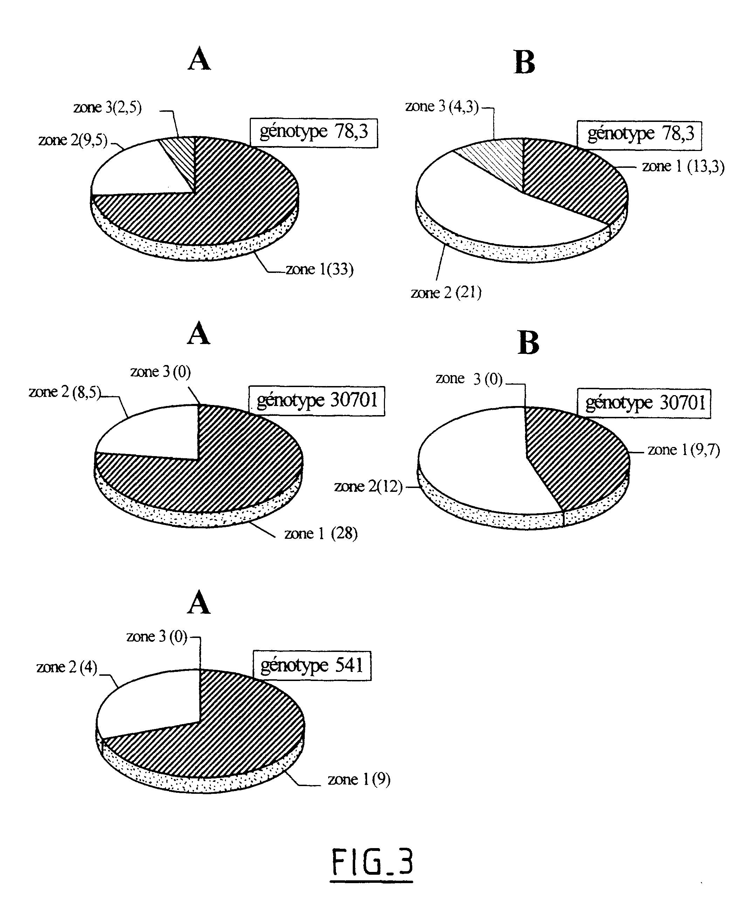

La figure 3 représente le taux d'initiation des tissus embryogènes (en % du nombre total d'aiguilles mises en culture) en fonction du site de prélèvement chez des bourgeons au stade pinceau, de plantes somatiques de *Picea abies* âgées de 52 (A) jours à 67 (B) jours depuis la graine.

### Figure 4 :

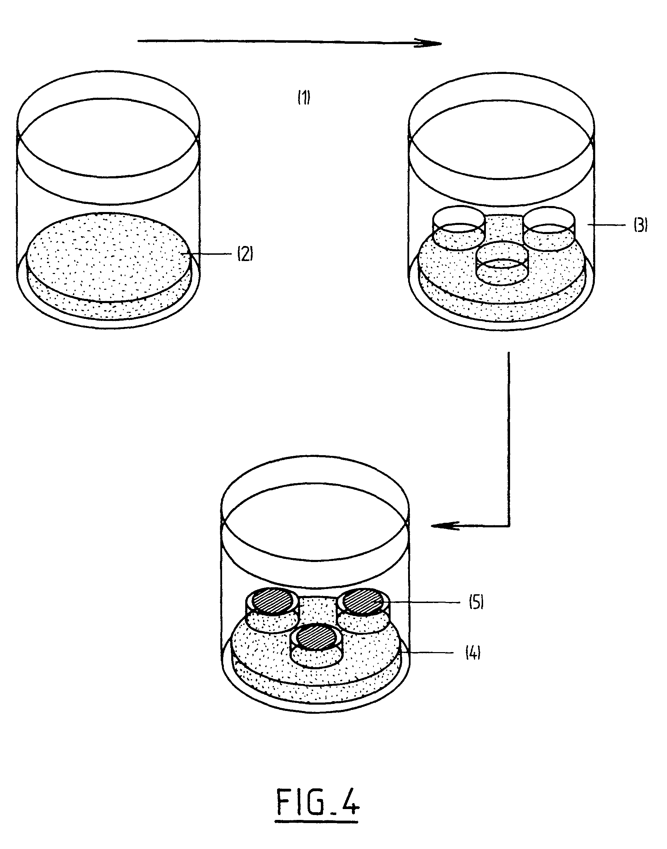

La figure 4 représente les éléments du procédé de co-culture en milieu liquide.

Sur cette figure :
(1) correspond au récipient de culture,
(2) correspond au support d'immobilisation,
(3) correspond aux dispositifs représentés,
(4) correspond à la nourrice (suspension des tissus embryogènes immobilisés sur le support), et
(5) correspond aux cals non embryogènes.

### Figure 5 :

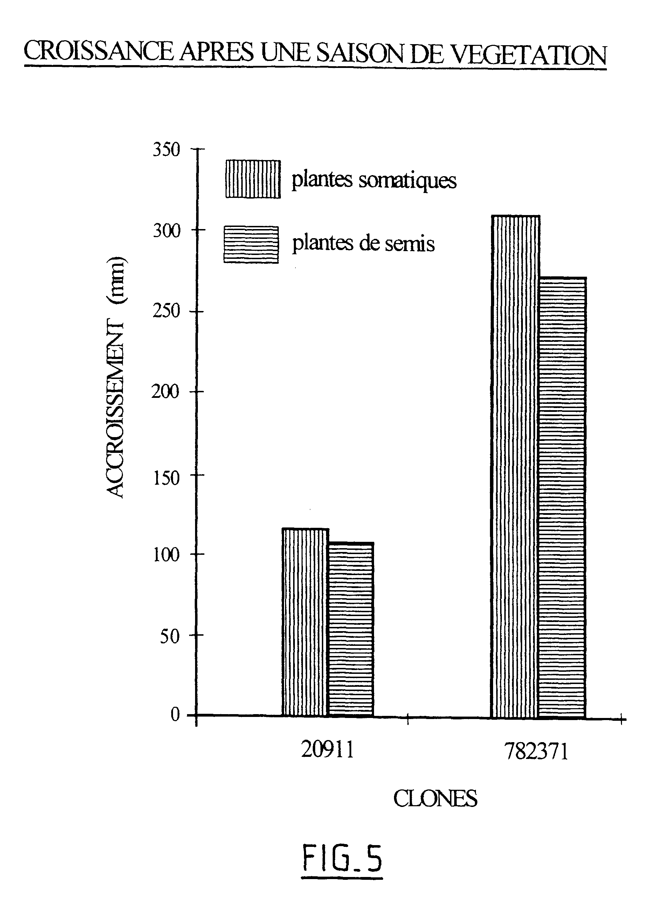

La figure 5 représente la comparaison de la croissance des plantes zygotiques (semis) avec celle de plantes somatiques régénérées à partir d'aiguilles prélevées sur des plantes âgées de 3 ans et de 22 ans après une saison de végétation.

### Figure 6 :

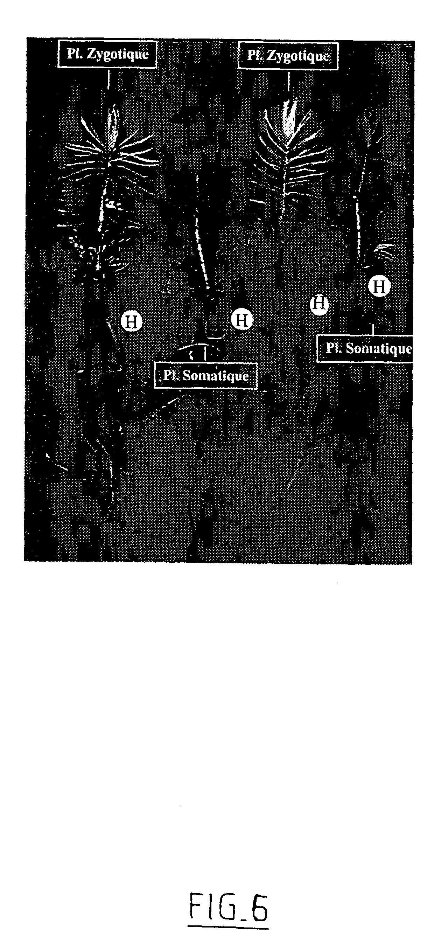

La figure 6 représente la comparaison entre des plantes zygotiques et des plantes somatiques, ces dernières étant régénérées par embryogenèse somatique à partir d'un arbre âgé (20 ans environ).

Sur la figure 6, H correspond à hypocotyle et C correspond à cotylédons ; les plantes somatiques expriment un niveau de juvénilité équivalent à celui des plantes zygotiques.

### I- INITIATION DE TISSUS EMBRYOGENES (ESM) A PARTIR D'AIGUILLES DE CONIFERES :

### A) CAS DE PICEA ABIES

### 1. Matériel végétal

### 1.1. Les clones :

On utilise des clones juvéniles âgés de quelques mois à 3 ans servant de contrôle juvénile et 10 des 40 meilleurs clones de provenances diverses sélectionnés par l'AFOCEL dans le cadre de ses programmes d'amélioration correspondant à des plantes originales âgées de 22 ans depuis la graine.

### 1.2. Etat physiologique des plants :

- Les clones âgés de 22 ans ont été maintenus dans un état végétatif par bouturages successifs tous les 3 ans.
- Les plants prélevés ont été réactivés soit naturellement (passage de l'hiver au printemps), soit artificiellement par exemple par forçage en serre chaude pendant 1 mois après une taille effectuée le printemps précédent.

Dans tous les cas, la plupart des plants prélevés portaient des bourgeons en cours de développement : bourgeons gonflés et bourgeons au moins en début d'épanouissement.

La caractérisation des bourgeons est représentée sur la figure 1.

Sur la figure 2, on a représenté la longueur moyenne des aiguilles par zone pour différents stades de développement des bourgeons de *Picea abies*.

### 1.3. Prélèvement des boutures et pré-conditionnement :

On a prélevé des rameaux latéraux de 10 cm de long contenant des bourgeons aux 3 stades de développement décrits sur la figure 1 et on a stocké à 4°C à l'obscurité pendant 5 à 15 jours.

### 2. Introduction du matériel végétal in vitro

### 2.1. Prélèvements et stérilisation des bourgeons :

Les bourgeons gonflés encore fermés sont prélavés à l'alcool éthylique 70% pendant 2 à 3 secondes.

Les bourgeons déjà ouverts sont immergés 10 min. dans de l'hypoclorite de calcium 7% avec agitation, puis rincés par 3 lavages à l'eau stérile

### 2.2. Prélèvements des explants et mise en culture :

Les aiguilles arrachées de l'axe principal sont posées par 20 sur le milieu de culture d'initiation, à l'obscurité et à une température moyenne de 23°C. Le volume du milieu est de 20 ml par boite de Pétri (12 cm de diamètre).

### 2.3. Milieu d'initiation :

**Tableau 1 :**

| Définition des milieux d'initiation des tissus embryogènes et des cals non embryogènes : | | | | |
|---|---|---|---|---|
| **N° DU MILIEU** | **SUBSTANCES DE CROISSANCE** | | | |
| | AUXINE (µM) | | CYTOKININE (µM) | |
| ① | NAA | (20) | BAP | (0,5) |
| ② | NAA | (20) | BAP | (5,0) |
| ③ | Picloram | (10) | BAP | (5,0) |
| ④ | Picloram | (10) | BAP | (0,5) |
| BAP, NAA et Picloram ont les significations indiquées précédemment. | | | | |

Les milieux contiennent 3% de saccharose; ils sont gélifiés par 0,7 g d'agar Bacto Difco et le pH est ajusté à 5,8 avant la stérilisation 20 min. à 1 atmosphère.

### 2.4. Repiquages tous les 21 jours sur un milieu frais.

### RESULTATS :

### INITIATION DE TISSUS EMBRYOGENES - INFLUENCE DU PICLORAM :

Les aiguilles sont prélevées sur des bourgeons en forte activité.

**Tableau 2 :**

| | | | | | |
|---|---|---|---|---|---|
| il représente le pourcentage des aiguilles de plantes somatiques ayant initié des tissus embryogènes, et ce, pour 3 clones : résultats de 3 répétitions dans le temps constituées chacune d'au moins 20 objets (aiguilles) répartis dans 3 boites de Pétri ; Ic = intervalles de confiance. ①, ②, ③ et ④ sont les milieux définis dans le tableau 1. | | | | | |

| **N° CLONES** | **TRAITEMENTS** | ① | ② | ③ | ④ |
|---|---|---|---|---|---|
| **Clone 78.3** | Aiguilles 51 jrs | 0,0 | 0,0 | 1,7 | 6,3 |
| | IC> | 0,0 | 0,0 | 9,3 | 33,0 |
| | Aiguilles 187 jrs | 0,0 | 0,0 | 0,0 | 5,0 |
| | IC > | 0,0 | 0,0 | 0,0 | 27,5 |
| **Clone 30701** | Aiguilles 51 jrs | 0,0 | 0,0 | 5,4 | 10,0 |
| | IC> | 0,0 | 0,0 | 28,4 | 50,0 |
| | Aiguilles 187 jrs | 0,0 | 0,0 | 0,0 | 4,0 |
| | IC > | 0,0 | 0,0 | 0,0 | 16,0 |
| **Clone 541.1** | Aiguilles 51 jrs | 0,0 | 0,0 | 0,0 | 6,1 |
| | IC > | 0,0 | 0,0 | 0,0 | 14,6 |
| | Aiguilles 187 jrs | - | - | - | - |
| | IC> | - | - | - | - |

Les rendements embryogènes des tissus embryogènes stabilisés obtenus à partir d'aiguilles prélevées sur 3 génotypes sont présentés au tableau 2.

Les rendements embryogènes obtenus à partir d'aiguilles prélevées sur des plantes cultivées *in vitro* âgées de 51 et 187 jours sont améliorés par un traitement au Picloram pour les trois génotypes testés (tableau 3). Le génotype 541 présente les résultats les plus faibles.

### INITIATION DE TISSUS EMBRYOGENES - STABILISATION ET ZONES DE PRELEVEMENT DES AIGUILLES :

La figure 3 représente l'initiation des tissus embryogènes (en % du nombre total d'aiguilles mises en culture) en fonction du site de prélèvement chez des bourgeons au stade pinceau, de plantes somatiques de *Picea abies* âgées de 51 jours (A) et de 67 jours.

Pour tous les génotypes, les zones 1 et 2 sont les plus réactives : (aucune différence significative n'ayant été notée entre les traitements, ceux-ci ont été confondus). Le méristème n'a jamais initié de tissus embryogènes dans les conditions testées.

### INITIATION-STABILISATION DE TISSUS EMBRYOGENES - DEVELOPPEMENT DES BOURGEONS ET ZONES DE PRELEVEMENT DES AIGUILLES :

**Tableau 3 :**

| **Paramètres** | **Stade I** | | **Stade II** | | **Stade III** | |
|---|---|---|---|---|---|---|
| | ① | ③ | ① | ③ | ① | ③ |
| **20911 AGE 3 ans** Bourgeons initiants des ESM stabilisés | 0/6 | 4/8 | 0/6 | 4/10 | 0/7 | 0/10 |
| Aiguilles initiants des ESM stables | 0/280 | 9/398 | 0/235 | 7/338 | 0/203 | 0/242 |
| **20911 AGE 2 ans** Bourgeons initiants des ESM stabilisés | 0/2 | 4/4 | 2/2 | 3/4 | 0/2 | 0/2 |
| Aiguilles initiants des ESM | 0/86 | 64/205 | 20/84 | 8/136 | 0/51 | 0/43 |
| ① et ③ sont les milieux définis dans le tableau 1. | | | | | | |

Les plantes somatiques âgées de trois ans étaient dans leur deuxième année de végétation en pépinière et ont été prélevées à partir de 47 bourgeons. Aucune différence n'a été notée concernant le comportement des bourgeons issus de plantes débourrées en serre ou à l'extérieur ; en conséquence, les deux séries d'expériences ont été regroupées.

Quelles que soient les conditions de culture, les stades 1 et 2 des bourgeons sont les plus réactifs. Près de 80% des bourgeons mis en culture initient des tissus embryogènes. Sur le total des bourgeons introduits 17% ont différencié des tissus embryogènes stabilisés. Ce sont les aiguilles issues des zones 1 et 2 qui sont les plus réactives ; c'est-à-dire que ce sont les aiguilles en élongation et les plus jeunes. Aucune structure embryogène n'a été observée ni sur les aiguilles de la zone 3, ni à partir du méristème . Sur les milieux d'induction, on note une nécrose très rapide de ce type d'aiguilles.

### INITIATION DE TISSUS EMBRYOGENES - CLONES AGES DE 22 ANS :

Pour les dix génotypes testés, deux types de comportement ont été observés :
- les génotypes dont les explants forment des nodules à l'origine pour certains de structures embryogènes. Certains de ces nodules initient des tissus embryogènes. Ce sont les clones 782371, 782498 et 783018 ;
- les génotypes dont les explants mis en culture ne forment que des cals non embryogènes. Ce sont les clones 770059, 770065, 780359, 780361, 782376 et 782179. Ce sont les clones réfractaires à l'embryogenèse somatique dans les conditions testées ;

L'ensemble des résultats est résumé dans le tableau 6. Pour trois clones, le pourcentage d'explants présentant des nodules est de l'ordre de 40%. Ce pourcentage est inférieur à celui noté pour les plantes somatiques. Quelques nodules ont donné naissance à des tissus embryogènes, après 3 à 4 mois de culture.

Seule la co-culture sur milieu solide de tissus embryogènes régénérés à partir des clones 782371, 783018 a été réalisée avec des tissus embryogènes stabilisés d'un autre clone. Elle a permis la stabilisation des tissus embryogènes des clones âgés de 22 ans depuis la graine et la régénération d'embryons somatiques cotylédonaires à partir de ceux-ci.

**Tableau 4 :**

| INITIATION DE TISSUS EMBRYOGENES A PARTIR DE BOURGEONS DE CLONES D'EPICEA AGES DE 22 ANS | | | | | | |
|---|---|---|---|---|---|---|
| **CLONE** | **Nbre moyen de nodules/bourgeons** | | | **Bourgeons avec ESM** | | |
| | Stade I | Stade II | Stade III | Stade I | Stade II | Stade III |
| 783271 | 69,75 | 39 | 21,75 | 2/4 | 1/4 | 0/4 |
| 782490 | 58,75 | 28,75 | 21,5 | 1/4 | 0/4 | 0/4 |
| 783018 | 34,75 | 17,75 | 12,75 | 1/4 | 0/4 | 0/4 |
| 770059 | 0 | 0 | 0 | 0/4 | 0/4 | 0/4 |
| 770065 | 0 | 0 | 0 | 0/4 | 0/4 | 0/4 |
| 780359 | 0 | 0 | 0 | 0/4 | 0/4 | 0/4 |
| 780754 | 0 | 0 | 0 | 0/4 | 0/4 | 0/4 |
| 780361 | 0 | 0 | 0 | 0/4 | 0/4 | 0/4 |
| 782376 | 0 | 0 | 0 | 0/4 | 0/4 | 0/4 |
| 782179 | 0 | 0 | 0 | 0/4 | 0/4 | 0/4 |

Les résultats ont été reproduits 3 fois pour le 782371 et le 783018 par des personnes différentes.

### B) CAS DU PINUS PINASTER

Des bourgeons fasciculaires de Pin maritime ont été prélevés au début de leur développement après réactivation. Le 1/3 supérieur des bourgeons dont la taille était d'environ 5 mm a été introduit *in vitro* selon le procédé décrit pour l'épicéa.

Sur les 4 semis expérimentés, 2 ont développé des ESM. Pour le premier, un bourgeon sur 12 a développé des ESM, pour l'autre 2 bourgeons sur 12. Il est à noter que les bourgeons, dont l'accroissement de longueur après réactivation excédait 10 mm, n'étaient pas réactifs.

Les ESM régénérés à partir de ces semis ont été co-cultivés en présence de ESM stabilisé de pin maritime selon le procédé décrit plus haut. Les ESM placés sur un milieu de maturation se développent en embryons "pré-cotylédonaires".

### II - INITIATION DE TISSUS EMBRYOGENES (ESM) A PARTIR DE CALS NON EMBRYOGENES (CNE) :

### A) CAS DE PICEA ABIES

Dans toutes les conditions d'initiation précédemment décrites, les cals non embryogènes sont différenciés à partir des aiguilles introduites *in vitro* comme décrit précédemment. Des cals non embryogènes co-cultivés avec des tissus embryogènes peuvent initier dans certaines conditions des tissus embryogènes à partir desquels des embryons, puis des plantes somatiques ont été régénérées. Le système utilisé est décrit ci-dessous.

### ELEMENTS DU SYSTEME DE CO-CULTURE (Figure 4) :

- le récipient de culture est une boîte en polycarbonate fermée par un bouchon à visser, celui-ci étant dévissé d'un quart de tour pendant la durée de la culture, ledit récipient de culture contenant :
   . le support d'immobilisation des tissus embryogènes constitué d'une mousse en polyuréthane dont le nombre théorique de cellules ouvertes peut être compris entre 20 et 60 par 2,54 centimètres,
   . les chambres de culture Millicell™ (diamètre = 12 mm, H = 10 mm, pores de 0,45 µm) sont déposées sur le support d'immobilisation contenant les cals non embryogènes.
- le milieu de culture ;
- Gupta et Durzan (1986) liquide, utilisé couramment pour la culture des suspensions embryogènes, mais contenant de l'ANA et du Picloram comme décrits ci-dessus.

### METHODE DE CO-CULTURE :

### . Immobilisation des tissus embryogènes :

Trois cent cinquante (350) mg des tissus embryogènes ont été mis en suspension dans 25 ml de milieu Gupta et Durzan (1986) pendant 7 jours, dans le récipient de culture contenant une mousse de polyuréthane utilisée comme support de fixation des tissus embryogènes.

A la fin de la période de préculture de 7 jours, les tissus embryogènes sont presque en totalité immobilisés dans le support d'immobilisation, des amas de tissus embryogènes peuvent être également observés à la surface de la mousse.

### . Culture des cals non embryogènes :

Cinquante (50) mg des cals non embryogènes sont posés dans une chambre Millicell™ , trois chambres sont placées à la surface de la mousse contenant les tissus embryogènes immobilisés.

Le nombre de chambres Millicell™ par récipient de culture pourra être porté à 7, mais jamais plus. Il sera fonction de l'intensité de la croissance des tissus embryogènes. A chaque opération de multiplication, on cherche à reconstituer les unités de repiquage d'environ 50 mg, en gardant, si possible, un rapport de masse d'environ 2,5 entre les cals non embryogènes et les tissus embryogènes.

### . Fréquence des repiquages :

Les cals non embryogènes sont cultivés en présence de la même culture des embryogènes pendant 42 à 45 jours. Tous les 21 jours, la totalité du milieu liquide du système est totalement renouvelée par le même milieu de culture frais.

Tous les 42-45 jours, le support d'immobilisation de polyuréthane contenant les tissus embryogènes immobilisés est renouvelé.
. Surface du support d'immobilisation : 113 cm2.
. Volume du milieu de culture : 25 ml.
. Température : 20 à 22°C.
. Lumière : 24 h obscurité.
. Humidité : non contrôlée.
. Agitation : plateau agitant rotateur : 95 tours par minute.

### RESULTATS :

### 1) L'INFLUENCE DE LA CO-CULTURE SUR LA CROISSANCE DES CALS NON EMBRYOGENES :

Après 21 jours, la production de biomasse des cals non embryogènes témoins (cultivés dans le système sur le support d'immobilisation de polyuréthanne sans tissus embryogènes immobilisés) est de 1,2 fois à 1,8 fois plus grande que la biomasse produite par les cals non embryogènes en co-culture. Après la fin du premier cycle de culture (42 jours), les différences sont encore plus grandes : le poids frais gagné par le témoin est de 1,7 fois à 2,3 fois supérieur à celui atteint par les cals non embryogènes en co-culture.

En d'autres termes, la co-culture en présence de tissus embryogènes limite le développement des cals non embryogènes.

En 3 semaines, les cals non embryogènes co-cultivés peuvent remplir toute la cavité de la chambre de culture et ce qui permet leur division en 3 parties. Les cals non embryogènes paraissent alors plus friables que sur milieu solide.

Après 6 semaines, des amas de grandes cellules transparentes peuvent être observés. Ils forment des glomérules plus dures que l'ensemble des cals, qui contiennent également des zones nécrotiques.

### 2) OBSERVATION DES PREMIERS TISSUS EMBRYOGENES : APRES 3 MOIS DE CO-CULTURE :

Aucun tissu embryogène n'a été observé pour les différents témoins testés. Dès lors, seuls les résultats concernant les co-cultures sont présentés dans le tableau 7. Après trois mois de culture, la formation des premiers tissus embryogènes est visible. Pour les deux conditions testées, au moins un cal par conteneur a initié des tissus embryogènes. Ce phénomène est reproductible.

**Tableau 8 :**

| Initiation de tissus embryogènes à partir de cals non embryogènes issus d'un génotype (Q40). | | | | | |
|---|---|---|---|---|---|
| REPETITION | Milieu | Nombre de Millicell™ au départ | Nombre de Millicell™ en final | Nombre de Millicell™ avec ESM | ESM induit/ nombre de Millicell™ au départ |
| R1 | ①/1 | 3 | 6 | 3 | 3/3(100%) |
| | ③/1 | 3 | 5 | 2 | 2/3(67%) |
| R2 | ①/1 | 18 | 35 | 7 | 7/18(38%) |
| | ③/1 | 9 | 12 | 2 | 2/9(22%) |
| R3 | ①/1 | 18 | 42 | 9 | 9/18(50%) |
| | ③/1 | 9 | 21 | 4 | 4/9(44%) |

Une quatrième répétition dans le temps a permis d'observer des ESM dans 4 Millicell sur 16.

### 3) OBTENTION D'EMBRYONS COTYLEDONAIRES A PARTIR DES DIFFERENTES SOUS-LIGNEES INITIEES PAR CO-CULTURE :

A titre d'exemple, les 4 tissus embryogènes obtenus à partir du clone Q40 sont aptes à former des embryons cotylédonaires de bonne qualité. Comme il a été mentionné précédemment, les rendements sont très variables d'une lignée à une autre.

**Tableau 6 :**

| Taux de multiplication et rendements en embryons cotylédonaires par gramme de matière fraîche des différentes sous-lignées régénérées par co-culture à partir du génotype Q40. Deux lignées par condition d'induction (CIN et CIP) ont été testées. | | | |
|---|---|---|---|
| Milieu d'induction | Lignée | Taux de multiplication | Embryons cotylédonaires / g matière fraîche |
| **CIN** | 1 | 2,4 | 50 |
| | 2 | 2,2 | 44 |
| **CIP** | 1 | 5,2 | 42 |
| | 2 | 4,8 | 172 |

Le pourcentage de germination des embryons est de l'ordre de 80% pour l'ensemble des lignées et plusieurs centaines de plantes de ce clone Q40 sont actuellement en croissance en pépinière.

### 4) COMPORTEMENT DES PLANTES SOMATIQUES REGENEREES A PARTIR D'AIGUILLES :

A titre d'illustration, on compare sur la figure 5 le comportement de plantes zygotiques (semis) à celui de plantes somatiques régénérées à partir d'aiguilles prélevées sur des plantes âgées de 3 ans et de 22 ans.

Dans tous les cas étudiés, le développement des plantes somatiques (figure 5), après une saison de végétation, est au moins équivalent à celui des semis. Ces résultats indiquent que le procédé permet de régénérer des plantes à vigueur juvénile à partir d'épicéas âgés de 22 ans depuis la graine.

### B) CAS DE PINUS RADIATA

Obtention d'ESM par co-culture selon le procédé décrit pour l'Epicéa.

Des bourgeons terminaux de semis âgés de 2 ans (provenance Portugaise) et de boutures de 2 ans réalisées à partir d'arbres âgés de 20 ans depuis la graine (provenance inconnue, plantation française) ont été prélevés. Ces tissus sont prélevés dans le 1/3 supérieur des bourgeons terminaux réactivés ayant subis un accroissement maximum de 10 mm depuis leur reprise d'activité. Ils ont été utilisés pour produire du cal non embryogène selon la procédure décrite pour Picea abies.

Les cals non embryogènes ont été mis en co-culture comme décrit précédemment à propos de Picea abies mais la culture nurse était des ESM de Pinus pinaster.

Des ESM ont été obtenus puis pré-amplifiés par co-culture sur un milieu solide à la fois pour le semis et les boutures d'arbres âgés. A titre d'exemple, 2/8 cals non embryogènes issus de l'arbre âgé ont différenciés des ESM. Les ESM issus de semis ont régénérés des embryons cotylédonaires ; les ESM issus de l'arbre âgé ont été transférés sur un milieu de maturation.

### III - ESTIMATION DE L'IDENTITE DES PLANTS REGENERES :

### A) CAS DE L'EPICEA

*Une première estimation de l'indice de similitude de Jaccard* a été réalisée pour comparer le niveau de similitude entre les profils RAPD obtenus à partir de l'ADN extrait des plantes somatiques régénérées à partir d'un arbre d'épicéa âgé de 22 ans depuis la graine et cet arbre âgé.

Les profils RAPD ont été obtenus de façon reproductible en s'assurant des meilleures conditions expérimentales : les amorces étaient des décamètres de type Opéron fournis par la firme BIOPROBE (OPG15, OPG1, OPB18, OPJ17), la concentration en MgCl2 dans le tampon de 1,8mM. 45 cycles d'amplification ont été utilisés et la température d'hybridation était de 37°C. Les bandes considérées sur le gel avaient une densité optique relative supérieure à 0,05 après une coloration standardisée des gels d'agarose au bromure d'éthidium.

L'analyse des profils RAPD avec 4 amorces indique une similitude moyenne de 80 % entre les profils de la plante âgée de 22 ans et les plantes somatiques régénérées à partir d'aiguilles prélevées sur le même génotype.

Le niveau de similitude est considéré suffisant pour conclure que les plantes somatiques régénérées à partir d'aiguilles sont bien des clones de la plante mère âgée de 22 ans depuis la graine.

### B) CAS DE PINUS RADIATA

L'origine des tissus embryogènes régénérés à partir de deux génotypes âgés de 3 ans a été confirmée, notamment par analyse microsatellite.

## Revendications

1. Utilisation de bourgeons en développement ou d'explants de bourgeons en développement de gymnospermes, notamment de conifères en développement, suite à une reprise de développement naturelle ou non après un arrêt de croissance naturel ou provoqué, pour l'initiation de tissus embryogènes et de cals non embryogènes, les aiguilles étant prélevées en dessous du méristème, et au-dessus des premières aiguilles en arrêt d'élongation, et plus particulièrement dans les 2/3 supérieurs de la pousse en élongation, et plus particulièrement dans le 1/3 supérieur de la pousse en élongation.

2. Utilisation de bourgeons ou d'explants de bourgeons selon la revendication 1, lesquels bourgeons ou explants sont prélevés sur la période s'étendant de la reprise du développement des bourgeons jusqu'à l'arrêt de croissance de ceux-ci.

3. Utilisation de bourgeons ou d'explants de bourgeons selon l'une des revendications 1 ou 2, dans laquelle les explants de bourgeons sont des aiguilles ou des écailles en cours d'élongation prélevées sur l'axe caulinaire en élongation.

4. Utilisation selon l'une des revendications 1 à 2, dans laquelle les conifères sont des gymnospermes appartenant à l'ordre des coniférales, et plus particulièrement aux familles des Pinaceae, Cupressaceae, Taxaceae, Taxodiaceae et plus particulièrement aux genres : Abies, Pinus, Picea, Tsuga, Pseudotsuga, Thyua, Juniperus, Larix, Sequoia.

5. Utilisation selon l'une des revendications 1 à 4, dans laquelle les explants ou bourgeons sont prélevés sur la période s'étendant de la reprise d'activité des bourgeons jusqu'à l'arrêt de croissance, et plus particulièrement du gonflement des bourgeons jusqu'à l'élongation de l'axe caulinaire portant le méristème, laquelle élongation est comprise d'environ 0,01 mm à environ 10 mm, et plus particulièrement d'environ 0,05 mm à environ 10 mm.

6. Utilisation selon l'une des revendications 1 à 5, dans laquelle le prélèvement est effectué sur des bourgeons choisis parmi des bourgeons végétatifs terminaux ou latéraux en arrêt de croissance suite à des conditions notamment climatiques naturelles ou artificielles, puis soumis à des conditions de reprise de développement, naturelles ou artificielles.

7. Utilisation selon l'une des revendications 1 à 6, dans laquelle les bourgeons sont prélevés sur de l'épicéa et ont la forme d'ananas ou de pinceau arrondi.

8. Utilisation de bourgeons ou d'explants de bourgeons selon l'une quelconque des revendications 1 à 7, prélevés sur des gymnospermes juvéniles ou matures et, notamment sur des individus âgés d'environ 51 jours à environ 22 ans ou plus depuis la graine, et particulièrement d'environ 67 jours à environ 22 ans ou plus depuis la graine, et notamment d'environ 2 ans à environ 22 ans ou plus depuis la graine.

9. Utilisation de bourgeons ou d'explants de bourgeons selon l'une quelconque des revendications 1 à 7, dans laquelle les bourgeons végétatifs sont prélevés sur les plants âgés d'environ 51 jours à environ 22 ans ou plus depuis la graine, notamment d'environ 67 jours à environ 22 ans ou plus depuis la graine, et notamment d'environ 2 ans à environ 22 ans ou plus depuis la graine et, plus particulièrement, sur des boutures de ces derniers, et plus avantageusement sur les boutures de ceux-ci obtenus par bouturages réitérés tous les trois ans pendant 22 ans au plus à partir d'un semi âgé de 4 ans.

10. Utilisation selon l'une des revendications 1 à 9, dans laquelle les rameaux sur lesquels sont prélevés les bourgeons sont traités par le froid et notamment soumis à des températures allant d'environ 15°C à environ 0°C pendant environ 10 à environ 20 jours à la lumière ou à l'obscurité, et plus particulièrement soumis à des températures allant d'environ 10°C à environ 5°C pendant environ 15 à environ 5 jours à l'obscurité.

11. Procédé d'initiation de tissus embryogènes et de cals non embryogènes de gymnospermes, notamment de conifères, par mise en culture de bourgeons en développement ou d'explants de bourgeons, notamment d'aiguilles, en développement de gymnospermes, notamment de conifères en développement, suite à une reprise de développement naturelle ou non après un arrêt de croissance naturel ou provoqué, les aiguilles étant prélevées en dessous du méristème, et au-dessus des premières aiguilles en arrêt d'élongation, et plus particulièrement dans les 2/3 supérieurs de la pousse en élongation, et plus particulièrement dans le 1/3 supérieur de la pousse en élongation, sur ou dans un milieu d'initiation contenant des substances de croissance utilisées isolément ou en combinaison, plus particulièrement des auxines, des cytokinines, des gibbérellines et plus avantageusement des auxines et des cytokinines.

12. Procédé de préparation de tissus embryogènes à partir des cals non embryogènes obtenus par mise en culture de bourgeons en développement ou d'explants de bourgeons en développement de gymnospermes, notamment de conifères en développement, suite à une reprise de développement naturelle ou non après un arrêt de croissance naturel ou provoqué, les aiguilles étant prélevées en dessous du méristème, et au-dessus des premières aiguilles en arrêt d'élongation, et plus particulièrement dans les 2/3 supérieurs de la pousse en élongation, et plus particulièrement dans le 1/3 supérieur de la pousse en élongation, comprenant les étapes suivantes :
- établissement d'une suspension de tissus embryogènes réactifs provenant avantageusement de tissus embryogènes de gymnospermes et,
- co-culture des tissus non embryogènes avec les tissus embryogènes en suspension ou avantageusement immobilisés sur un support sur lequel les tissus pourront s'immobiliser soit par effet mécanique, soit par effet électrochimique ; les tissus non embryogènes étant séparés des tissus embryogènes par une membrane dont la taille moyenne des pores est comprise d'environ 0,20 µm à environ 5,0 µm, avantageusement d'environ 0,23 à environ 0,5 µm.

13. Procédé selon la revendication 12, dans lequel l'étape d'immobilisation a lieu dans un milieu liquide, notamment un milieu de prolifération classique, et l'étape de co-culture a lieu dans un milieu liquide avantageusement différent du susdit milieu liquide utilisé pour l'immobilisation, et notamment un milieu d'induction classique.

14. Tissus embryogènes tels qu'obtenus par la mise en oeuvre du procédé selon l'une des revendications 11 à 13.

15. Procédé de régénération de plantes à partir de tissus embryogènes tels qu'obtenus selon l'une des revendications 11 à 13.

## Patentansprüche

1. Verwendung von sich entwickelnden Knospen oder von Explantaten sich entwickelnder Knospen von Gymnospermen, insbesondere von sich entwickelnden Koniferen, nach einer Wiederaufnahme der natürlichen oder nicht natürlichen Entwicklung nach einem natürlichen oder herbeigeführten Wachstumsstillstand, zur Initiierung embryogener Gewebe und nichtembryogener Kalli, wobei die Nadeln an der Unterseite des Meristems, und oberhalb der ersten Nadeln in Verlängerungsstillstand, und vornehmlich in den oberen 2/3 des sich verlängernden Sprosses, und vornehmlich im oberen 1/3 des sich verlängernden Sprosses entnommen werden.

2. Verwendung von Knospen oder von Explantaten von Knospen nach Anspruch 1, wobei die Knospen oder Explantate während des Zeitraums, der sich von der Wiederaufnahme der Entwicklung der Knospen bis zu ihrem Wachstumsstillstand erstreckt, entnommen werden.

3. Verwendung von Knospen oder von Explantaten von Knospen nach einem der Ansprüche 1 oder 2, wobei die Explantate von Knospen sich verlängernde Nadeln oder Schuppen sind, die entlang der sich verlängernden cauloiden Achse entnommen wurden.

4. Verwendung nach einem der Ansprüche 1 bis 2, wobei die Koniferen Gymnospermen sind, welche zu der Ordnung der Coniferales und insbesondere zu den Familien der Pinaceae, Cupressaceae, Taxaceae, Taxodiaceae und insbesondere zu den Gattungen Abies, Pinus, Picea, Tsuga, Pseudotsuga, Thyua, Juniperus, Larix, Sequoia gehören.

5. Verwendung nach einem der Ansprüche 1 bis 4, wobei die Explantate oder Knospen während des Zeitraums der sich von der Wiederaufnahme der Aktivität der Knospen bis zu ihrem Wachstumsstillstand erstreckt, entnommen werden, und vornehmlich vom Anschwellen der Knospen bis zur Verlängerung der das Meristem tragenden cauloiden Achse, wobei die Verlängerung zwischen etwa 0,01 mm und etwa 10 mm, und vornehmlich zwischen etwa 0,05 mm und etwa 10 mm liegt.

6. Verwendung nach einem der Ansprüche 1 bis 5, wobei die Entnahme von Knospen erfolgt, die aus aufgrund insbesondere natürlicher oder künstlicher klimatischer Bedingungen im Wachstumsstillstand befindlichen, terminalen oder lateralen vegetativen Knospen ausgewählt werden, und die dann natürlichen oder künstlichen Bedingungen zur Wiederaufnahme der Entwicklung unterworfen werden.

7. Verwendung nach einem der Ansprüche 1 bis 6, wobei die Knospen von der Fichte entnommen werden und die Form einer Ananas oder eines abgerundeten Pinsels haben.

8. Verwendung von Knospen oder Explantaten von Knospen nach einem der Ansprüche 1 bis 7, entnommen von juvenilen oder reifen Gymnospermen, und insbesondere von Individuen mit einem Alter von etwa 51 Tagen bis etwa 22 Jahren oder mehr ab Samen, insbesondere mit einem Alter von etwa 67 Tagen bis etwa 22 Jahren oder mehr ab Samen, und vornehmlich mit einem Alter von etwa 2 Jahren bis etwa 22 Jahren oder mehr ab Samen.

9. Verwendung von Knospen oder Explantaten von Knospen nach einem der Ansprüche 1 bis 7, wobei die vegetativen Knospen von Pflanzen entnommen werden mit einem Alter von etwa 51 Tagen bis etwa 22 Jahren oder mehr ab Samen, insbesondere mit einem Alter von etwa 67 Tagen bis etwa 22 Jahren oder mehr ab Samen, und insbesondere mit einem Alter von etwa 2 Jahren bis etwa 22 Jahren oder mehr ab Samen, und vornehmlicher von den Stecklingen der letzteren, und vorteilhaftererweise von ihren Stecklingen, erhalten durch wiederholtes Setzen der Stecklinge alle drei Jahre während 22 Jahren oder mehr ab einem Saatalter von 4 Jahren.

10. Verwendung nach einem der Ansprüche 1 bis 9, wobei die Zweige, von denen die Knospen entnommen werden, kältebehandelt werden, und insbesondere Temperaturen von etwa 15°C bis etwa 0°C über etwa 10 bis etwa 20 Tage im Licht oder in der Dunkelheit ausgesetzt werden, und vornehmlich Temperaturen von etwa 10°C bis etwa 5°C über etwa 15 bis etwa 5 Tage in der Dunkelheit ausgesetzt werden.

11. Verfahren zur Initiierung von embryogenen Geweben und von nichtembryogenen Kalli von Gymnospermen, insbesondere von Koniferen, durch Kultivierung von sich entwickelnden Knospen oder Explantaten von Knospen, insbesondere von Nadeln, in der Entwicklung von Gymnospermen, insbesondere von sich entwickelnden Koniferen, nach einer Wiederaufnahme der natürlichen oder nicht natürlichen Entwicklung nach einem natürlichen oder herbeigeführten Wachstumsstillstand, wobei die Nadeln an der Unterseite des Meristems und oberhalb der ersten Nadeln in Verlängerungsstillstand, vornehmlich in den oberen 2/3 des sich verlängemden Sprosses und vornehmlich im oberen 1/3 des sich verlängernden Sprosses entnommen werden, in oder auf ein Initiierungsmedium, welches einzeln oder kombiniert verwendete Wachstumssubstanzen enthält, vornehmlich Auxine, Cytokinine, Gibberelline, und vorteilhaftererweise Auxine und Cytokinine.

12. Verfahren zur Herstellung von embryogenen Geweben aus nichtembryogenen Kalli, durch Kultivierung von sich entwickelnden Knospen oder Explantaten von sich entwickelnden Knospen von Gymnospermen, insbesondere von sich entwickelnden Koniferen, nach einer Wiederaufnahme der natürlichen oder nicht natürlichen Entwicklung nach einem natürlichen oder herbeigeführten Wachstumsstillstand, wobei die Nadeln an der Unterseite des Meristems und oberhalb der ersten Nadeln in Verlängerungsstillstand, und vornehmlich in den oberen 2/3 des sich verlängernden Sprosses und vornehmlich im oberen 1/3 des sich verlängernden Sprosses, entnommen werden, wobei das Verfahren die folgenden Schritte umfasst:
- Etablierung einer Suspension reaktiver embryogener Gewebe, welche vorteilhafterweise von embryogenen Geweben von Gymnospermen stammen, und
- Co-Kultivierung der nichtembryogenen Gewebe mit den embryogenen Geweben die suspendiert oder vorteilhafterweise auf einem Träger immobilisiert sind, auf dem sich die Gewebe entweder durch mechanische Wirkung oder durch elektrochemische Wirkung immobilisieren lassen; wobei die nichtembryogenen Gewebe von den embryogenen Geweben durch eine Membran getrennt sind, deren Porendurchschnittsgröße zwischen etwa 0,20 µm und etwa 5,0 µm, vorteilhafterweise etwa zwischen 0,23 und etwa 0,5 µm liegt.

13. Verfahren nach Anspruch 12, wobei der Schritt der Immobilisierung in einem flüssigen Medium, insbesondere einem klassischen Proliferationsmedium stattfindet, und der Schritt der Co-Kultivierung in einem flüssigen Medium stattfindet, welches vorteilhafterweise unterschiedlich zu dem vorher erwähnten, für die Immobilisierung verwendeten flüssigem Medium, und insbesondere ein klassisches Induktionsmedium ist.

14. Embryogene Gewebe, wie sie durch den Einsatz des Verfahrens nach einem der Ansprüche 11 bis 13 erhältlich sind.

15. Verfahren zur Regeneration von Pflanzen ausgehend von embryogenen Geweben, wie sie nach einem der Ansprüche 11 bis 13 erhältlich sind.

## Claims

1. The use of developing buds or developing bud explants from gymnosperms, in particular from developing conifers, following a natural or non-natural development resumption, after a natural or induced growth arrest, for the initiation of embryogenic tissues and of non-embryogenic calli, the needles being taken below the meristem, and above the first needles in elongation arrest, and more particularly in the upper 2/3 of the elongating shoot, and more particularly in the upper 1/3 of the elongating shoot.

2. The use of buds or of bud explants according to claim 1, said buds or explants being taken during the period ranging from the development resumption of the buds to the growth arrest of the latter.

3. The use of buds or of buds explants according to claim 1 or 2, wherein the bud explants are elongating needles or scales taken from the elongating stem axis.

4. The use according to any of claims 1 to 2, wherein the conifers are gymnosperms belonging to the Coniferale order, and more particularly to the Pinaceae, Cupressaceae, Taxaceae, Taxodiaceae families, and more particularly to the Abies, Pinus, Picea, Tsuga, Pseudotsuga, Thyua, Juniperus, Larix, Sequoia genera.

5. The use according to any of claims 1 to 4, wherein the explants or the buds are taken during the period ranging from the activity resumption of the buds to the growth arrest, and more particularly from the swelling of the buds to the elongation of the meristem holding stem axis, said elongation ranging from about 0.01 mm to about 10 mm, and more particularly from about 0.05 mm to about 10 mm.

6. The use according to any of claims 1 to 5, wherein the buds which are taken are selected from the terminal or lateral vegetative buds in growth arrest, following natural or artificial conditions, in particular climatic conditions, and exposed to natural or artificial development resumption conditions.

7. The use according to any of claims 1 to 6, wherein the buds are taken from spruce and are in the form of a pineapple or of a rounded paintbrush.

8. The use of buds or bud explants according to any of claims 1 to 7, taken from juvenile or mature gymnosperms, and in particular from individuals aged from about 51 days to about 22 years or more from seed, and particularly from about 67 days to about 22 years or more from seed, and in particular from about 2 years to about 22 years or more from seed.

9. The use of buds or bud explants according to any of claims 1 to 7, wherein the vegetative buds are taken from plants aged from about 51 days to about 22 years or more from seed, in particular from about 67 days to about 22 years or more from seed, and in particular from about 2 years to about 22 years or more from seed and, more particularly, from clones of the latter, and more advantageously from clones of the latter obtained by repeated cloning every three years during 22 years at the most from 4 years old seedlings.

10. The use according to any of claims 1 to 9, wherein the branches on which the buds are taken are treated by cold, and are in particular subjected to temperatures ranging from about 15°C to about 0°C during about 10 to about 20 days in the light or in the obscurity, and are more particularly subjected to temperatures ranging from about 10°C to about 5°C during about 15 to about 5 days in the obscurity.

11. A process for the initiation of embryogenic tissues and non-embryogenic calli from gymnosperms, in particular conifers, by culturing developing buds or developing bud explants, in particular needles, from gymnosperms, in particular from developing conifers, following a natural or non-natural development resumption after a natural or induced growth arrest, the needles being taken below the meristem, and above the first needles in elongation arrest, and more particularly in the upper 2/3 of the elongating shoot, and more particularly in the upper 1/3 of the elongating shoot, onto or into an initiation medium containing growth substances used alone or in combination, more particularly auxins, cytokinins , gibberelins and more advantageously auxins and cytokinins.

12. A process for preparing embryogenic tissues from the non-embryogenic calli obtained from culturing developing buds or developing bud explants, in particular needles, from gymnosperms, in particular from developing conifers, following a natural or non-natural development resumption after a natural or induced growth arrest, the needles being taken below the meristem, and above the first needles in elongation arrest, and more particularly in the upper 2/3 of the elongating shoot, and more particularly in the upper 1/3 of the elongating shoot, comprising the following steps:
- establishing a suspension of reactive embryogenic tissues advantageously from gymnosperm embryogenic tissues and,
- co-culturing non-embryogenic tissues with embryogenic tissues in suspension or advantageously immobilized on a support on which the tissues can be immobilized either by a mechanical effect or by an electrochemical effect; the non-embryogenic tissues being set apart from the embryogenic tissues by a membrane, the pores average size of which ranges from about 0.20 µm to about 5.0 µm, advantageously from about 0.23 to about 0.5 µm.

13. A process according to claim 12, wherein the immobilization step takes place in a liquid medium, in particular in a classical proliferation medium, and the co-culture step takes place in a liquid medium advantageously different from the abovementioned liquid medium used for immobilization, in particular in a classical induction medium.

14. Embryogenic tissues such as obtained by applying the process according to any of claims 11 to 13.

15. A process for regenerating plants from embryogenic tissues such as obtained according to any of claims 11 to 13.
